# EUROPEAN PATENT SPECIFICATION

(11) **EP 1 104 546 B1**
(45) Date of publication and mention of the grant of the patent: **01.03.2006**
(21) Application number: 99938662.6
(22) Date of filing: 06.08.1999
(51) Int. Cl.: G01N 33/543, B05D 7/24

(54) **DEVICE AND PROCESS FOR INVESTIGATING CHEMICAL INTERACTIONS**
VERFAHREN UND EINRICHTUNG ZUM UNTERSUCHEN DER CHEMISCHEN INTERAKTIONEN
DISPOSITIF POUR L'ANALYSE D'INTERACTIONS CHIMIQUES ET SON PROCEDE D'UTILISATION

(30) Priority: 14.08.1998 NL 1009871
(43) Date of publication of application: 06.06.2001
(73) Proprietor: HOLLAND BIOMATERIALS GROUP B.V., NL-7522 NB Enschede (NL)
(72) Inventor: TERLINGEN, Johannes, Gijsbertus, Antonius, NL-6373 VT Landgraaf (NL); ENGBERS, Gerardus, Henricus, Maria, NL-7577 MB Oldenzaal (NL)
(74) Representative: Duxbury, Stephen
(86) International application number: PCT/NL1999/000504
(87) International publication number: WO 2000/010012

(56) References cited:
- EP-A- 0 104 608
- EP-A- 0 806 250
- WO-A-97/38801
- US-A- 5 055 316
- US-A- 5 266 309
- US-A- 5 627 079
- US-A- 5 723 219
- PATENT ABSTRACTS OF JAPAN vol. 098, no. 002, 30 January 1998 (1998-01-30) & JP 09 257797 A (SEKISUI CHEM CO LTD), 3 October 1997 (1997-10-03)

## Description

The present invention relates to a device for investigating reactions between interactive chemical and/or biological species, to a process for providing such a device, and to a process for investigating chemical and/or biological interactions, for example biomolecular interactions, utilizing such a device.

Under chemical and/or biological interactions is also understood chemical and/or biological reactions.

Interactions of specific compounds with solid surfaces play a crucial role in chemical and biological phenomena and areas including analysis techniques such as RIA's, ELISA's.

For investigating and sensing surface interactions a 'sensitive' surface is required.

To study real time surface interactions several techniques are available such as ellipsometry, reflectometry and surface plasmon resonance spectroscopy (SPR). These techniques have in common that they use the reflectance of light, generated by a laser, to analyze the growth or desintegration of a layer of for instance biological molecules at a surface.

For these techniques, a reflecting surface is necessary. In the case of SPR, a surface comprising a free electron metal for example gold is most frequently used.

In order to utilize this technique for investigating other interactions, besides the interaction of (bio)molecules with free electron metal surfaces, the free electron surfaces have been modified, for instance, by the adsorption of bio-molecules such as proteins and the coating thereof with polymeric layers in a solvent cast or spin coat procedures.

Methods have also been developed to provide gold surfaces with specific chemical groups for the immobilization of proteins, which surfaces are subsequently utilized for studying the interactions with other (biological) substances such as antibody-antigen interactions.

Methods for generating SPR sensor surfaces include arranging an organic surface onto a gold layer by means of a wet chemistry procedure such as solvent casting or spin coating before carrying out a plasma etching procedure.

A further method includes adsorption of a chemical functional surfactant, by means of a wet chemistry procedure, on the surface to be modified and the subsequent immobilization of the surfactant by a plasma such as an argon plasma, so called plasma immobilization.

WO 97/38801 discloses a process for the deposition of a reactive functional group on a surface of a solid substrate. The substrate may be a polymer, ceramic, metal, silanized metal, carbon, fabric, glass, silanized glass, wood, cellulose, ceramic, composite, rubber, rubber film, polymer, polymer film, semiconductor, paper or hydrogel. The reactive functional group may be an isothiocyanate, cyanide, benzene, acetate, mercaptan, glycidyl ether, ether, chloroformate, methyl sulfide, phenyl sulfone, phosphonic dichloride, trimethylsilane, triethoxysilane, carbon-halogen, acid halide, acid anhydride, sulfhydryl, phosphide, carbonyl or carboxylic acid. The interaction with the surface was subsequently analysed by ESCA.

US-A-5,627,079 discloses a method for fluorination of a surface by gas face fluorination or plasma deposition. The surface to be fluorinated is selected from the group consisting of polymeric, ceramic and metalic materials. The metalic materials may be selected from a group consisting of gold, nikkel, copper, aluminum, steel alloys, ferrous and non-ferrous alloys. Thus, prior to functionalization the surface of the substrate is fluorinated.

Disadvantages of these known techniques include the lack of stability of the functional surface layers.

An object of the present invention is to provide an improved device for investigating the reactions between interactive chemical species.

According to a first aspect of the present invention there is provided a device according to any of the claims 1 to 6.

The device according to the present invention provides a good attachment of the plasma deposited layer, a good stability thereof and a device exhibiting good sensitivity, whereby the substrate is provided with a functional layer, the functionality of which can be provided by groups such as amine, carboxylic acid, hydroxyl, acid chloride, isocyanate, aldehyde, anhydride, epoxide, and thiol groups for example.

According to a second aspect of the present invention, there is provided a process according to any of the claims 7 to 16 for providing the device according to the present invention.

Since a functional group layer is plasma deposited, control over the deposition thereof can be accurately carried out, whereby very thin layers can be deposited thus providing very sensitive devices, without the need for firstly arranging an organic layer by wet chemical methods on the substrate before any further investigation can be carried out.

The process according to the present invention provides a good controllability.

In contrast to processes for providing sensor devices, wherein layers are arranged on a substract by wet chemical processes which are often time consuming, difficult to carry out, and often result in undesirably thick layers exhibiting a subsequent lack of sensitivity if a great deal of care in not applied, the process according to the present invention is extremely flexible to work and easy to effect and offers a good cost efficiency.

Plasma deposition procedures involve the deposition of organic species from the plasma phase on a substrate. For instance by applying a (volatile) monomer as the gas phase an organic layer the structure of which resembles the corresponding polymer can be deposited. By applying a (volatile) monomer that possesses a chemical functionality a chemical functional polymeric layer can be obtained.

The plasma may be deposited from a monomer preferably being selected from the group consisting essentially of:
- unsaturated monomers; acrylic acid, allyl amine, allyl isocyanate, allyl mercaptan, methacrylic acid, allyl alcohol, allyl acetate, allyl acetic acid, allyl glycidyl ether, 3 allyloxy, 1-2 propanediol, vinyl acetate, acrylic acid halides,
- saturated monomers; alcohols such as methanol, ethanol propanol, acids such as propionic acid, acetic acid and the like, formaldehyde, propionic aldehyde, glutardialdehyde, aminoethane, aminoethanol, ethylene oxide, acetone methane, ethane, propane and the like, whereby the substrate is provided with the corresponding functionality.

Apart from the plasma deposition of saturated and unsaturated monomers, a functionality can be created in situ, i.e. in the plasma layer, by means of rearrangements of (cyclic) monomers or reaction between a mixture of plasma gases for example, whereafter this in-situ created functionality can be deposited.

Surfaces with a high surface energy, such as metal surfaces in general, may give rise to a rapid surface hydrophobisation due to contamination of the surface by species from its environment. This surface contamination may be disastrous for further surface modification for instance with respect to the stability of the final surface. Therefore this surface contamination should be prevented as much as possible by storing the surfaces in an inert atmosphere and reduction of the time between surface preparation and modification or the surface needs to be cleaned before modification. Plasma etching offers an excellent method for this cleaning. Plasma cleaning is fast and is a clean process in itself since it does not involve the use of organic solvent or substantial amounts of reagents that may have adverse effects on the environment. For the present invention it is advantageous to include an in situ plasma cleaning step of the substrate before the actual modification by plasma deposition.

The plasma deposited layer comprises one or more sulphur compounds, for example thiols, sulfides and/or.disulfides, i.e. in the form of mercaptoacetic acid, 2-mercaptopropionic acid, 3-mercaptopropionic acid, 1-mercaptopropenol, 2-mercaptoethanol and the like, preferably diallylsulfide, since gold is chosen as the substrate whereby an improved stability is provided.

According to a further aspect of the present invention there is provided a pocess and use of a device according to any of the claims 1 to 6 for investigating the interaction of chemical and/or biological species, for example real time surface interactions, according to claims 17-19.

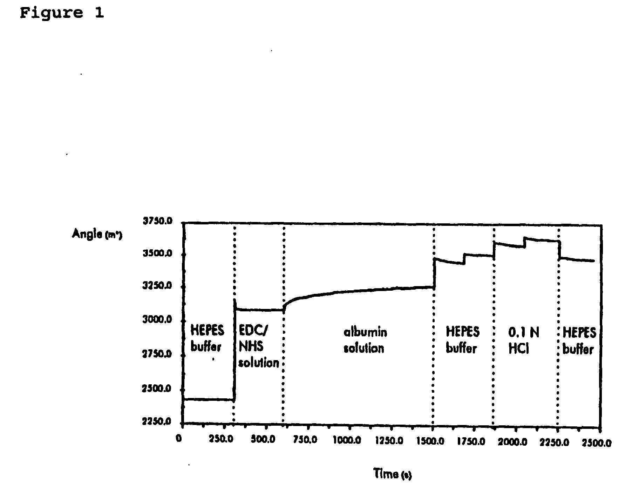

The invention will now be further clarified by way of the following examples, with reference to figure 1 which graphically shows the immobilization of albumins onto a COOK disk as carried out in example 12.

### Example 1

### Preparation of carboxylic acid functional gold surfaces.

Gold coated glass discs (60) were placed in the central position of the plasma reactor which consisted of a glass tubes (1 = 150 cm, o= 10 cm) with three electrodes positioned at the outside of the glass tube with the powered electrode in the center and two grounded electrodes positioned at 30 cm distance on both sides of the powered electrode. The electrodes were connected to an RF-generator (13.56 MHz, ENI ACG-3, ENI Power Systems) through a matching network (ENI Matchwork 5) and a matching network control unit (ENI TH-1000, ENI). The generator was controlled by a timer (Apple Ile computer with a time control program).

The reactor was evacuated to a pressure less than 0,1 Pa (0.001 mbar) by a rotary pump (DUO 004 B, Pfeifer) which was equipped with a filter (ONF 025, Pfeifer) to prevent oil back streaming. The pressure was measured by a pressure gauge (Baratron 628A01MDE, MKS Instruments) and read from a display module (PR4000, MKS Instruments). An air flow of 5 sccm/min resulting in a pressure of about 2 Pa (0.12 mbar) was established for 5 minutes after which the discs were treated with a dynamic air plasma (85 W) for 1 minute at the same flow conditions. Air flow was controlled by a mass flow controller (type 1259 + PR3000 control unit, MKS Instruments). After the plasma treatment the air flow was continued for 2 minutes and then stopped and an acrylic acid flow was established through the reactor via a direct monomer inlet resulting in a pressure of about 3 Pa (0.03 mbar). To prevent the acrylic acid to reach the pump after leaving the reactor, the acrylic acid flow was bypassed through a cold trap that was cooled with liquid nitrogen. The temperature of the acrylic acid in the storage container was room temperature. After two minutes the surfaces were treated with 5 pulses of an acrylic acid plasma at a discharge power of 75 (W), the pulses being separated from each other by 30 seconds of acrylic acid flow through the reactor. After the final pulse the surface were exposed to 2 additional minutes of acrylic acid flow whereupon the acrylic acid flow was stopped and the reactor was brought to atmospheric pressure with air.

### Example 2

### Preparation of amine functional surfaces

Gold coated glass discs (60) were placed in the plasma reactor as described in example 1. The reactor was evacuated to a pressure of less than 0.05 mbar and an air flow of 5 sccm/min was established for 5 minutes whereupon the discs were treated with a dynamic air plasma (85 W) for 1 minute at the same flow conditions. Then air flow was stopped and an allyl amine flow 7 Pa (0.07 mbar) was established through the reactor the temperature of the monomer storage container was 36°C. After two minutes the surfaces were treated with 10 pulses of an allyl amine plasma at a discharge power of 75 W separated from each other by 10 seconds of allyl amine flow through the reactor. After the final pulse the surfaces were exposed to 2 additional minutes of allyl amine flow after which the allyl amine flow was stopped and the reactor was brought to atmospheric pressure with air.

### Example 3

Gold coated substrates (6) were placed in the plasma reactor (see example 2) between the cold electrode on the gas inlet side of the reactor and the hot electrode. The reactor was evacuated to a pressure less than 0,5 Pa (0.005 mbar) and an air flow of 5 sccm was established through the reactor. After 2 minutes of air flow the substrates were treated with a dynamic air plasma (5 sccm, 85 W) for 1 minute and subsequently exposed to an air flow of 5 sccm for 10 minutes again. Then the air flow was stopped and after evacuation of the reactor, an allylamine flow at a pressure of 9.5 Pa (0.095 mbar) was established through the reactor. After two minutes allylamine flow the substrates were exposed to ten pulses of I second of an allylamine plasma at a discharge power of 85 W, the pulses being separated by ten seconds allylamine flow. After the final allylamine plasma pulse the allylamine flow was continued for 2 minutes after which the flow was discontinued, the reactor was evacuated and subsequently brought to atmospheric pressure with air. Following, the surfaces were analyzed for carbon, oxygen, nitrogen and gold by X-ray photo-electron spectroscopy, of which the results are shown in the table below. Also surfaces that were rinsed with water for 1 hr and subsequently dried were analyzed by XPS.

**Table 1**

| element | surface composition (at%0) | |
|---|---|---|
| | before rinsing | after rinsing |
| C | 65.4 | 62.4 |
| O | 10.3 | 10.5 |
| N | 17.5 | 13.6 |
| Au | 6.8 | 13.4 |

### Example 4

Gold coated substrates (6) were placed in the plasma reactor (see example 2) between the cold electrode on the gas inlet side of the reactor and the hot electrode. The reactor was evacuated to a pressure of less than 0.5 Pa (0.005 mbar) and an argon flow of 5 sccm was established through the reactor. After 2 minutes of argon flow the substrates were treated with a dynamic argon plasma (5 sccm, 85 W) for 1 minute and subsequently exposed to an argon flow of 5 sccm for 10 minutes again. Then the argon flow was stopped and after evacuation of the reactor, an allylamine flow at a pressure of 0.095 mbar was established through the reactor. After two minutes allylamine flow the substrates were exposed to ten pulses of 1 second of an allylamine plasma at a discharge power of 85 W, the pulses being separated by ten seconds allylamine flow. After the final allylamine plasma pulse the allylamine flow was continued for 2 minutes after which the flow was discontinued, the reactor was evacuated and subsequently brought to atmospheric pressure with air.

### Example 5

Gold coated substrates (6) were placed in the plasma reactor (see example 2) between the cold electrode on the gas inlet side of the reactor and the hot electrode. The reactor was evacuated to a pressure of less than 0,5 Pa (0.005 mbar) and an air flow of 5 sccm was established through the reactor. After 2 minutes of air flow the substrates were treated with a dynamic air plasma (5 sccm, 85 W) for 1 minute and subsequently exposed to an air flow of 5 sccm for 10 minutes again. Then the air flow was stopped and after evacuation of the reactor, an allylamine flow at a pressure of 9.5 Pa (0.095 mbar) was established through the reactor. After two minutes allylamine flow the substrates were exposed to five pulses of 1 second of an allylamine plasma at a discharge power of 170 W, the pulses being separated by ten seconds allylamine flow, followed by five pulses of an allylamine plasma at a discharge power of 85 W, again the pulses being separated by ten seconds allylamine flow. After the final allylamine plasma pulse the allylamine flow was continued for 2 minutes after which the flow was discontinued, the reactor was evacuated and subsequently brought to atmospheric pressure with air. Following, the surfaces were analyzed for carbon, oxygen, nitrogen and gold by X-ray photo-electron spectroscopy, of which the results are shown in the table below.

**Table 2.**

| element | surface composition (atomic %) |
|---|---|
| C | 62.8 |
| O | 9.8 |
| N | 20.8 |
| Au | 6.6 |

### Example 6

Gold coated substrates (6) were placed in the plasma reactor (see example 2) between the cold electrode on the gas inlet side of the reactor and the hot electrode The reactor was evacuated to a pressure of less than 0.5 Pa (0.005 mbar) and an air flow of 5 sccm was established through the reactor. After 2 minutes of air flow the substrates were treated with a dynamic air plasma (5 sccm, 85 W) for 1 minute and subsequently exposed to an air flow of 5 Sccm for 10 minutes again. Then the air flow was stopped and after evacuation of the reactor, a mixed flow of allylamine and octadiene (66 v% allylamine) at a pressure of 5.5 Pa (0.055 mbar) was established through the reactor. After two minutes allylamine/octadiene flow the substrates were exposed to ten pulses of 1 second of an allylamine/octadiene plasma at a discharge power of 85 W, the pulses being separated by ten seconds allylamine/octadiene flow. After the final-plasma pulse the allylamine/octadiene flow was continued for 2 minutes after which the flow was discontinued, the reactor was evacuated and subsequently brought to atmospheric pressure with air. Following, the surfaces were analyzed for carbon, oxygen, nitrogen and gold by X-ray photo-electron spectroscopy, of which the results are shown in the table below.

**Table 3**

| element | surface composition (atomic %) |
|---|---|
| C | 73.1 |
| O | 7.9 |
| N | 11.7 |
| Au | 7.3 |

### Example 7

Gold coated substrates (6) were placed in the plasma reactor (see example 2) between the cold electrode on the gas inlet side of the reactor and the hot electrode. The reactor was evacuated to a pressure of less than 0,5 Pa (0.005 mbar) and an air flow of 5 sccm was established through the reactor. After 2 minutes of air flow the substrates were treated with a dynamic air plasma (5 sccm, 85 W) for 1 minute and subsequently exposed to an air flow of 5 sccm for 10 minutes again. Then the air flow was stopped and after evacuation of the reactor, a mixed flow of allylamine and diallylsulfide (66 v% allylamine) at a presssure of 6,5 Pa (0.065 mbar) was established through the reactor. After two minutes allylamine/diallylsulfide flow the substrates were exposed to ten pulses of 1 second of an allylamine/diallylsulfide plasma at a discharge power of 85 W, the pulses being separated by ten seconds allylamine/diallylsulfide flow. After the final plasma pulse the allylamine/diallylsulfide flow was continued for 2 minutes after which the flow was discontinued, the reactor was evacuated and subsequently brought to atmospheric pressure with air.

Following, the surfaces were analyzed for carbon, oxygen, nitrogen and gold by X-ray photo-electron spectroscopy, of which the results are shown in the table below. Also surfaces that were rinsed with water for 1 hr and subsequently dried were analyzed by XPS.

**Table 4**

| element | surface composition (at%0) | |
|---|---|---|
| | before rinsing | after rinsing |
| C | 73.4 | 68.3 |
| O | 4.4 | 5.3 |
| N | 8.3 | 9.0 |
| S | 13.3 | 16.5 |
| Au | 0.7 | 0.9 |

### Example 8

Gold coated substrates (6) were placed in the plasma reactor (see example 2) between the cold electrode on the gas inlet side of the reactor and the hot electrode. The reactor was evacuated to a pressure of less than 0.5 Pa (0.005 mbar) and an air flow of 5 sccm was established through the reactor. After 2 minutes of argon flow the substrates were treated with a dynamic argon plasma (5 sccm, 85 W) for 1 minute and subsequently exposed to an argon flow of 5 sccm for 10 minutes again. Then the argon flow was stopped and after evacuation of the reactor, a mixed flow of allylamine and diallylsulfide (66 v% allylamine) at a pressure of 6,5 Pa (0.065) mbar was established through the reactor. After two minutes allylamine/diallylsulfide flow the substrates were exposed to ten pulses of 1 second of an allylamine/diallylsulfide plasma at a discharge power of 85 W, the pulses being separated by ten seconds allylamine/diallylsulfide flow. After the final plasma pulse the allylamine/diallylsulfide flow was continued for 2 minutes after which the flow was discontinued, the reactor was evacuated and subsequently brought to atmospheric pressure with air.

### Example 9

Gold coated substrates (6) were placed in the plasma reactor (see example 2) between the cold electrode on the gas inlet side of the reactor and the hot electrode. The reactor was evacuated to a pressure of less than 0,5 Pa (0.005 mbar) and an air flow of 5 sccm was established through the reactor. After 2 minutes of air flow the substrates were treated with a dynamic air plasma (5 sccm, 85 W) for 1 minute and subsequently exposed to an air flow of 5 sccm for 10 minutes again. Then the air flow was stopped and after evacuation of the reactor to a pressure less than 0.5 Pa (0.005 mbar), a diallylsulfide flow at a pressure of 0.025 mbar was established through the reactor. After two minutes diallylsulfide flow the substrates were exposed to ten pulses of 1 second of an diallylsulfide plasma at a discharge power of 85 W, the pulses being separated from each other by ten seconds diallylsulfide flow. After the final diallylsulfide plasma pulse the diallylsulfide flow was continued for 1 minute after which the flow was discontinued and the reactor was evacuated to a pressure less than 0.1 Pa (0.001 mbar). Then an allylamine flow at a pressure of 9 Pa (0.090 mbar) was established through the reactor. After two minutes allylamine flow the substrates were exposed to ten pulses of 1 second of an allylamine plasma at a discharge power of 85 W, the pulses being separated by ten seconds allylamine flow. After the final allylamine plasma pulse the allylamine flow was continued for 2 minutes whereafter the flow was discontinued and the reactor was evacuated to a pressure less than 0.1Pa (0.001 mbar) and brought to atmospheric pressure with air.

Following, the surfaces were analyzed for carbon, oxygen, nitrogen sulphur and gold by X-ray photo-electron spectroscopy, of which the results are shown in the table below. Also surfaces that were rinsed with water for f hr and subsequently dried were analyzed by XPS.

**Table 5**

| element | surface composition (at%0) | |
|---|---|---|
| | before rinsing | after rinsing |
| C | 69.8 | 68.3 |
| O | 6.9 | 10.2 |
| N | 14.8 | 12.9 |
| S | 8.5 | 8.6 |
| Au | 0.0 | 0.0 |

### Example 10

Gold coated substrates (6) were placed in the plasma reactor (see example 2) between the cold electrode on the gas inlet side of the reactor and the hot electrode. The reactor was evacuated to a pressure of less than 0.5 Pa (0.005 mbar) and an argon flow of 5 sccm was established through the reactor. After 2 minutes of argon flow the substrates were treated with a dynamic argon plasma (5 sccm, 85 W) for 1 minute and subsequently exposed to an argon flow of 5 sccm for 10 minutes again. Then the argon flow was stopped and after evacuation of the reactor to a pressure less than 0,5 Pa (0.005 mbar), a diallylsulfide flow at a pressure of 2,5 Pa (0.025 mbar) was established through the reactor. After two minutes diallylsulfide flow the substrates were exposed to ten pulses of 1 second of an diallylsulfide plasma at a discharge power of 85 W, the pulses being separated from each other by ten seconds diallylsulfide flow. After the final diallylsulfide plasma pulse the diallylsulfide flow was continued for 1 minute after which the flow was discontinued and the reactor was evacuated to a pressure less than 0.1 Pa (0.001 mbar). Then an allylamine flow at a pressure of 9 Pa (0.090 mbar) was established through the reactor. After two minutes allylamine flow the substrates were exposed to ten pulses of 1 second of an allylamine plasma at a discharge power of 85 W, the pulses being separated by ten seconds allylamine flow. After the final allylamine plasma pulse the allylamine flow was continued for 2 minutes after which the flow was discontinued and the reactor was evacuated to a pressure less than 0,1 Pa (0.001 mbar) and brought to atmospheric pressure with air.

### Example 11

### Coupling of CMD onto amine functionalized gold surfaces.

Carboxymethyl cellulose (100 mg) was dissolved in 10 ml 0.05 M 2-(N-morpholino) ethanesulfonic acid after which 5 mg N-hydroxysuccinimid was added. After complete dissolution of this reagent 20 mg N-(3-dimethylaminopropyl)-N' ethylcarbodiimide was added. After 3 minutes activation, an amine functionalized gold surface was incubated with 1 ml of this carboxymethyl dextran solution for 2,5 hours. Then the surfaces were rinsed with phosphate buffered saline, and water and vacuum dried. The whole immobilization procedure was performed at room temperature.

In this example, carboxymethyldextran is used as a model compound for chemical functional group containing compounds in general including but not limited to dextrans including carboxymethyl dextran, carboxymethyl cellulose, mono- di- oligo- and polysaccharides, gum xanthan, carboxylate and amine dendrimers, and mono-, homo- and hetero-functional carboxylate polyethylene glycols and polyethylene oxide, polyethylene imine, polyacrylic acid, polyvinyl alcohol, etc.

The amount of these functional group containing compounds that is immobilized can be controlled by the reaction parameters such as reaction time, the concentration of the functional group containing compound and the ratio of coupling agent to functional group containing compound.

### Example 12

### Immobilization of albumin on a COOH-functionalizes sensing device.

A sensor device, that was COOH-functionalized by the plasma deposition method was used for the immobilization of albumin. During the immobilization procedure that was performed at 22.5°C the surface events were monitored by Surface Plasmon Resonance Spectroscopy of which the results are given in figure 1. After mounting the functionalized sensing device in the SPR apparatus, the sensing surface was incubated with 10 mM HEPES buffer for about 5 minutes. Then the HEPES buffer was exchanged for a EDC (20 mg/ml)-NHS (4 mg/ml) solution in water. After 5 minutes activation the EDC/NHS solution was exchanged for an albumin solution (2 mg/ml in 10 mM HEPES) and an immobilization time of 15 minutes was applied. Then the sensing surface was rinsed with HEPES buffer and the stability of the immobilized albumin in HEPES buffer was monitored for 3 minutes after which the rinsing procedure with HEPES buffer was repeated. To study the stability of the immobilized albumin in 0.1 N HCl the HEPES buffer was replaced by 0.1 HCl and the sensing surface was incubated in this solution for 3 minutes after which 0.1 N HCl was replaced for fresh 0.1 N HCl and the measurement was continued for 3 minutes. Then the surface was rinsed with 0.1 N HEPES buffer again an incubation of the sensing surface was proceeded in this buffer for a final 5 minutes.

The results show that upon activation of the sensing surface with EDC/NHS and subsequent immobilization of albumin and rinsing with HEPES buffer the response increases with about 700 milli-degrees indicating the immobilization of albumin on the COOH-functionalized sensing surface. Rinsing of the surface with 0.1 N HCl only resulted in a decrease of the signal of about 30 milli-degrees, showing that the albumin immobilization is very stable.

The invention is not limited to the above description; the requested rights are rather determined by the following claims.

## Claims

1. Device for investigating reactions between interactive chemical and/or biological species, said device comprising:
- a substrate and
- a plasma layer deposited on the substrate,
**characterized in that** the substrate in turn comprises a film of free electron metal consisting essentially of gold, and wherein the plasma layer deposited on the film of free electron metal comprises one or more sulphur compounds.

2. Device according to claim 1, wherein the plasma deposited layer, comprises one or more chemical and/or biological functional groups.

3. Device according to.claim 2, further comprising one or more wet chemically deposited layer(s), arranged on the plasma deposited layer.

4. Device according to any of the preceding claims wherein the plasma layers comprise one or more amine compounds.

5. Device according to claim 1-4, wherein the sulphur compound comprises thiols, sulfides and/or disulfides and preferably being diallyl sulfide.

6. Device according to claim 1-5, wherein the substrate consists essentially of gold.

7. Process for providing a device for investigating reactions between interactive chemical and biological species, said process comprising the steps of providing a preselected substrate, which substrate in turn comprises a film of free electron metal consisting essentially of gold and arranging a layer on the gold film by plasma deposition, which layer comprise one ore more sulphur Compounds*.*

8. Process according to claim 7 wherein the plasma layer is directly deposited onto the metal film arranged on the substrate.

9. Process according to claims 7 or 8 wherein plasma is deposited from a monomer/ oligomer/ polymer in gas form, preferably being a monomer, said monomer being saturated, partially saturated or unsaturated.

10. Process according to any of the claims 7-9 wherein the substrate is subjected to a pre-cleaning step comprising pre-treating the substrate by means of a plasma etching step before the plasma deposition step said pre-cleaning step preferably comprising pre-treatment with air plasma.

11. Process according to any of the claims 7-10 wherein the gas plasma is deposited under the following conditions:
- a discharge power of upto 5000 W, preferably upto 500 W,
- an exposure duration of upto 1000 s, preferably upto 100 s,
- a plasma gas flow of upto 10000 cm³/min, preferably upto 100 cm³/min,
- a pressure of upto 10⁵Pa (1 bar), preferably from between 0,1-5000Pa (0,001-50 mbar),
- a frequency covering DC, AC, RF, and the MW, preferably from between 2-60 Mhz.

12. Process according to claim 11 wherein the discharge power is pulsed to the plasma, the pulse discharges being separated by:
- upto 1000 s preferably upto 100 s.

13. Process according to claims 11 or 12 wherein the substrate is treated in an after-glow.

14. Process according to claims 12 or 13 wherein following pulse discharge, the substrate is after-treated with a pre-selected gas, which gas optionally comprises the one or more functional groups which have been plasma deposited.

15. Process for providing a device according to any of the preceding claims 7-14, suitable for investigating reactions between interactive bio/chemical species by means of surface plasmon resonance spectroscopy, said process comprising the steps of:
- preselecting a free electron metal substrate, which metal substrate is suitable for allowing investigation by surface plasmon resonance spectroscopy, arranging a preselected first functional group species on the free electron metal substrate by means of plasma deposition, which first functional group species protects the free electron metal substrate from a second functional group species whose interaction with the plasma deposited first functional group species can be investigated, thereby preventing undesirable interactions between the free electron metal substrate and the second functional group species, and which first functional group species provides a desired functionality for the second functional group species, and
- subsequently arranging a second functional group species on the plasma deposited layer of the first functional group species, whereafter interaction between the first and second functional group species layers, can be investigated by means of surface plasmon resonance spectroscopy.

16. Process according to claim 15, wherein before being exposed to the second functional group species, a bio/chemical functional layer is wet chemically arranged on the plasma deposited first functional group species layer, said wet chemically arranged functional layer being preselected for its specificity for the second functional group species and for the prevention of non specific interactions with the said second functional group species.

17. Process for investigating the interaction, for example real time surface interaction, of pre-determined chemical and/or biological species, comprising the steps of analyzing the interaction between the species arranged on a device according to any of the claims 1 to 6.

18. Use of a device according to any of the claims 1-6, for investigating the reaction between chemically interactive species, and especially for use in SPR.

19. Use of a device for investigating reactions between interactive bio/chemical species, by means of surface plasmon resonance spectroscopy, said device comprising a preselected free electron metal substrate, and a preselected, plasma deposited layer arranged on the free electron metal substrate, which plasma deposited functional group species is chosen for its attachment ability to the free electron metal substrate, and for its specificity to further functional group species, whereby the interaction therebetween is investigatable by means of surface plasmon resonance spectroscopy, wherein the pre-selected free electron metal substrate consists essentially of gold, and wherein the plasma deposited layer comprises one or more.sulphur compounds.

## Patentansprüche

1. Vorrichtung zur Untersuchung der Reaktionen zwischen interaktiven chemischen und/oder biologischen Spezies, wobei die Vorrichtung aufweist:
- ein Substrat und
- eine Plasmaschicht, die auf dem Substrat abgeschieden ist,
**dadurch gekennzeichnet, dass** das Substrat seinerseits einen Film aus Metall mit freien Elektronen, im Wesentlichen bestehend aus Gold, aufweist, und wobei die auf dem Film aus Metall mit freien Elektronen abgeschiedene Plasmaschicht eine oder mehrere Schwefelverbindungen enthält.

2. Vorrichtung nach Anspruch 1, wobei die abgeschiedene Plasmaschicht ein oder mehrere chemische und/oder biologische Funktionsgruppen enthält.

3. Vorrichtung nach Anspruch 2, weiterhin mit einer oder mehreren nass chemisch abgeschiedenen Schicht(en), die auf der abgeschiedenen Plasmaschicht angeordnet ist oder sind.

4. Vorrichtung nach einem der vorstehenden Ansprüche, wobei die Plasmaschichten ein oder mehrere Aminoverbindungen enthalten.

5. Vorrichtung nach Anspruch 1 bis 4, wobei die Schwefelverbindungen Thiole, Sulfide und/oder Disulfide enthalten und vorzugsweise Diallylsulfide sind.

6. Vorrichtung nach Anspruch 1 bis 5, wobei das Substrat im Wesentlichen aus Gold besteht.

7. Verfahren zum Schaffen einer Vorrichtung zum Untersuchen der Reaktionen zwischen interaktiven chemischen- und biologischen Spezies, wobei das Verfahren die Schritte aufweist: Vorsehen eines vorgewählten Substrats, wobei das Substrat seinerseits einen Film aus Metall mit freien Elektronen aufweist, das im Wesentlichen aus Gold besteht, und Anordnen einer Schicht auf dem Goldfilm durch Plasmaabscheidung, wobei die Schicht ein oder mehrere Schwefelverbindungen enthält.

8. Verfahren nach Anspruch 7, wobei die Plasmaschicht direkt auf den Metallfilm abgeschieden wird, der auf dem Substrat angeordnet ist.

9. Verfahren nach Anspruch 7 oder 8, wobei das Plasma aus einem Monomer/Oligomer/Polymer in Gasform abgeschieden wird, vorzugsweise einem Monomer, wobei das Monomer gesättigt, partiell gesättigt oder ungesättigt ist.

10. Verfahren nach einem der Ansprüche 7 bis 9, wobei das Substrat einem Vorreinigungsschritt unterzogen wird, mit Vorbehandeln des Substrats mittels eines Plasmaätzschrittes vor dem Plasmaabscheidungsschritt, wobei der Vorreinigungsschritt vorzugsweise eine Vorbehandlung mit Luftplasma umfasst.

11. Verfahren nach einem der Ansprüche 7 bis 10, wobei das Gasplasma unter den folgenden Bedingungen abgeschieden wird:
- Entladungsleistung von bis zu 5000 W, vorzugsweise bis zu 500 W,
- Belichtungsdauer bis zu 1000 s, vorzugsweise bis zu 100 s,
- Plasmagasstrom bis zu 10000 cm³/min, vorzugsweise bis zu 100 cm³/min,
- Druck bis zu 10⁵Pa (1 bar), vorzugsweise von 0,1-5000Pa (0,001-50 mbar),
- einer Frequnez, die DC, AC, RF und MW abdeckt, vorzugsweise zwischen 2-60 Mhz.

12. Verfahren nach Anspruch 11, wobei die Entladungsleistung auf das Plasma gepulst wird, wobei die Pulsentladungen getrennt sind durch:
- bis zu 1000 s, vorzugsweise bis zu 100 s.

13. Verfahren nach Anspruch 11 oder 12, wobei das Substrat in einem Nachglühen behandelt wird.

14. Verfahren nach Anspruch 12 oder 13, wobei auf die Pulsentladung folgend das Substrat mit einem vorgewählten Gas nachbehandelt wird, welches Gas wahlweise die eine oder mehrere Funktionsgruppen enthält, die plasmaabgeschieden worden sind.

15. Verfahren zum Schaffen einer Vorrichtung gemäß einem der vorstehenden Ansprüche 7 bis 14, das für die Untersuchung der Reaktionen zwischen interaktiven biologischen/chemischen Spezies mittels Oberfächenplasmon-Resonanzspektroskopie geeignet ist, wobei das Verfahren die Schritte aufweist:
- Vorwählen eines Metallsubstrats mit freien Elektronen, wobei das Metallsubstrat dafür geeignet ist, eine Untersuchung durch die Oberflächenplasmon-Resonanzspektroskopie zu ermöglichen, Anordnen einer ersten vorgewählten Funktionsgruppenspezies auf dem Metallsubstrat mit freien Elektronen mittels Plasmaabscheidung, wobei die erste Funktionsgruppenspezies das Metallsubstrat mit freien Elektronen vor einer zweiten Funktionsgruppenspezies schützt, deren Interaktion mit der plasmaabgeschiedenen, ersten Funktionsgruppenspezies untersucht werden kann, wodurch unerwünschte Interaktionen zwischen dem Metallsubstrat mit freien Elektronen und der zweiten Funktionsgruppenspezies verhindert werden und welche erste Funktionsgruppenspezies eine gewünschte Funktionalität für die zweite Funktionsgruppenspezies bereitstellt, und
- darauf folgendes Anordnen einer zweiten Funktionsgruppenspezies auf der plasmaabgeschiedenen Schicht der ersten Funktionsgruppenspezies, worauf die Interaktion zwischen den ersten und zweiten Funktionsgruppenspeziesschichten mittels Oberflächenplasmon-Resonanzspektroskopie untersucht werden kann.

16. Verfahren nach Anspruch 15, wobei vor dem Aussetzen der zweiten Funktionsgruppenspezies eine biologisch/chemische Funktionsschicht nass chemisch auf der plasmaabgeschiedenen, ersten Funktionsgruppenspeziesschicht angeordnet wird, wobei die nass chemisch angeordnete Funktionsschicht bezüglich ihrer Spezifität für die zweite Funktionsgruppenspezies und zur Verhinderung von nicht spezifischen Interaktionen mit der zweiten Funktionsgruppenspezies vorgewählt ist.

17. Verfahren zum Untersuchen der Interaktion beispielsweise der Echtzeitoberflächeninteraktion von vorbestimmten chemischen und/oder biologischen Spezies, mit den Schritten Analysieren der Interaktion zwischen den Spezies, die auf der Vorrichtung gemäß einem der Ansprüche 1 bis 6 angeordnet sind.

18. Verwenden einer Vorrichtung gemäß einem der Ansprüche 1 bis 6 zum Untersuchen der Reaktion zwischen chemisch interaktiven Spezies und insbesondere zur Verwendung in SPR.

19. Verwendung einer Vorrichtung zum Untersuchen der Reaktionen zwischen interaktiven biologischen/chemischen Spezies mittels Oberflächenplasmon-Resonanzspektroskopie, wobei die Vorrichtung aufweist: ein vorgewähltes Metallsubstrat mit freien Elektronen und eine vorgewählte plasmaabgeschiedene Schicht, die auf dem Metallsubstrat mit freien Elektroden angeordnet ist, wobei die plasmaabgeschiedene Funktionsgruppenspezies bezüglich ihres Anhaftvermögen an dem Metallsubstrat mit freien Elektronen und bezüglich ihrer Spezifität für weitere Funktionsgruppenspezies gewählt ist, wodurch die Interaktion zwischen diesen mittels Oberflächenplasmon-Resonanzspektroskopie untersuchbar ist, wobei das vorgewählte Metallsubstrat mit freien Elektronen im Wesentlichen aus Gold besteht und wobei die plasmaabgeschiedene Schicht eine oder mehrere Schwefelverbindungen enthält.

## Revendications

1. Dispositif pour étudier les réactions entre des espèces chimiques et/ou biologiques interactives, ledit dispositif comprenant :
- un substrat et
- une couche de plasma déposée sur le substrat,
**caractérisé en ce que** le substrat comprend à son tour un film de métal à électrons libres constitué essentiellement d'or, et dans lequel la couche de plasma déposée sur le film de métal à électrons libres comprend un ou plusieurs composés de soufre.

2. Dispositif selon la revendication 1, dans lequel la couche de plasma déposée comprend un ou plusieurs groupes fonctionnels chimiques et/ou biologiques.

3. Dispositif selon la revendication 2, comprenant en outre une ou plusieurs couches déposées chimiquement par voie humide, disposées sur la couche de plasma déposée.

4. Dispositif selon l'une quelconque des revendications précédentes, dans lequel les couches de plasma comprennent un ou plusieurs composés amine.

5. Dispositif selon les revendications 1 à 4, dans lequel le composé de soufre comprend des thiols, des sulfures et/ou disulfures et de préférence un sulfure de diallyle.

6. Dispositif selon la revendication 1 à 5, dans lequel le substrat est essentiellement constitué d'or.

7. Procédé pour fournir un dispositif pour étudier les réactions entre des espèces chimiques et biologiques interactives, ledit procédé comprenant les étapes consistant à fournir un substrat présélectionné, lequel substrat comprend à son tour un film de métal à électrons libres constitué essentiellement d'or, et à disposer une couche sur le film d'or par dépôt par plasma, laquelle couche comprend un ou plusieurs composés de soufre.

8. Procédé selon la revendication 7, dans lequel la couche de plasma est directement déposée sur le film de métal disposé sur le substrat.

9. Procédé selon la revendication 7 ou 8 dans lequel le plasma est déposé à partir d'un monomère/oligomère/polymère sous forme gazeuse, de préférence un monomère, ledit monomère étant saturé, partiellement saturé ou insaturé.

10. Procédé selon l'une quelconque des revendications 7 à 9, dans lequel le substrat est soumis à une étape de prénettoyage comprenant le prétraitement du substrat au moyen d'une étape de gravure par plasma avant l'étape de dépôt du plasma, ladite étape de prénettoyage comprenant de préférence un prétraitement avec du plasma dans l'air.

11. Procédé selon l'une quelconque des revendications 7 à 10, dans lequel le plasma gazeux est déposé dans les conditions suivantes :
- une puissance de décharge allant jusqu'à 5 000 W, de préférence jusqu'à 500 W,
- une durée d'exposition allant jusqu'à 1 000 s, de préférence jusqu'à 100 s,
- un écoulement gazeux de plasma allant jusqu'à 10 000 cm³/min, de préférence jusqu'à 100 cm³/min,
- une pression allant jusqu'à 10⁵ Pa (1 bar), de préférence comprise entre 0,1 et 5 000 Pa (0,001 à 50 mbar),
- une fréquence couvrant le courant continu, le courant alternatif, les fréquences radioélectriques et les moyennes fréquences, de préférence comprise entre 2 et 60 MHz.

12. Procédé selon la revendication 11, dans lequel la puissance de décharge est donnée au plasma, les décharges étant séparées de :
- jusqu'à 1 000 s, de préférence jusqu'à 100 s.

13. Procédé selon la revendication 11 ou 12, dans lequel le substrat est traité dans une rémanence.

14. Procédé selon la revendication 12 ou 13, dans lequel à la suite de la décharge, le substrat est post-traité avec un gaz présélectionné, lequel gaz comprend éventuellement le ou les groupes fonctionnels ayant été déposés par plasma.

15. Procédé pour fournir un dispositif selon l'une quelconque des revendications précédentes 7 à 14, approprié pour étudier les réactions entre les espèces bio/chimiques interactives au moyen d'une spectroscopie de résonance plasmonique de surface, ledit procédé comprenant les étapes consistant à :
- présélectionner un substrat métallique à électrons libres, lequel substrat métallique est approprié pour permettre une étude par spectroscopie de résonance plasmonique de surface, disposer une première espèce de groupe fonctionnel présélectionnée sur le substrat métallique à électrons libres au moyen d'un dépôt par plasma, laquelle première espèce de groupe fonctionnel protège le substrat métallique à électrons libres d'une seconde espèce de groupe fonctionnel dont leur interaction avec la première espèce de groupe fonctionnel déposée par plasma peut être étudiée, empêchant ainsi les interactions non souhaitables entre le substrat métallique à électrons libres et la seconde espèce de groupe fonctionnel, et laquelle première espèce de groupe fonctionnel fournit une fonctionnalité souhaitée pour la seconde espèce de groupe fonctionnel, et
- disposer ultérieurement une seconde espèce de groupe fonctionnel sur la couche déposée par plasma de la première espèce de groupe fonctionnel, et par la suite l'interaction entre les couches des première et deuxième espèces de groupe fonctionnel peut être étudiée au moyen d'une spectroscopie de résonance plasmonique de surface.

16. Procédé selon la revendication 15, dans lequel avant l'exposition à la seconde espèce de groupe fonctionnel, une couche fonctionnelle bio/chimique est disposée chimiquement par voie humide sur la couche de la première espèce de groupe fonctionnel déposée par plasma, ladite couche fonctionnelle disposée chimiquement par voie humide étant présélectionnée pour sa spécificité pour la seconde espèce de groupe fonctionnel et pour la prévention des interactions non spécifiques avec ladite seconde espèce de groupe fonctionnel.

17. Procédé pour étudier l'interaction, par exemple, l'interaction des surfaces en temps réel, d'espèces chimiques et/ou biologiques prédéterminées, comprenant les étapes consistant à analyser l'interaction entre les espèces disposées sur un dispositif selon l'une quelconque des revendications 1 à 6.

18. Utilisation d'un dispositif selon l'une quelconque des revendications 1 à 6, pour étudier la réaction entre les espèces interactives chimiquement, et spécialement pour une utilisation en SPR.

19. Utilisation d'un dispositif pour étudier les réactions entre des espèces bio/chimiques interactives, au moyen d'une spectroscopie de résonance plasmonique de surface, ledit dispositif comprenant un substrat métallique à électrons libres présélectionné, et une couche déposée par plasma présélectionnée disposée sur le substrat métallique à électrons libres, laquelle espèce de groupe fonctionnel déposée par plasma est choisie pour sa capacité de liaison au substrat métallique à électrons libres, et pour sa spécificité pour d'autres espèces de groupes fonctionnels, de sorte que leur interaction peut être étudiée au moyen d'une spectroscopie de résonance plasmonique de surface, dans laquelle le substrat métallique à électrons libres présélectionné est essentiellement constitué d'or, et dans laquelle la couche déposée par plasma comprend un ou plusieurs composés de soufre.
